(19) Europäisches Patentamt

European Patent Office

Office européen des brevets

(11) **EP 3 901 791 A1**

(12) **EUROPEAN PATENT APPLICATION**

(43) Date of publication:
**27.10.2021 Bulletin 2021/43**

(21) Application number: **21169416.1**

(22) Date of filing: **20.04.2021**

(51) Int Cl.:
**G06F 17/18** (2006.01)   **G06N 3/04** (2006.01)
**G06N 5/00** (2006.01)   **G16H 50/00** (2018.01)
**G06F 21/00** (2013.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.04.2020   US 202063012447 P**

(71) Applicant: **Replica Analytics
Ottawa, Ontario K1P 5J6 (CA)**

(72) Inventors:
• **EL EMAM, Khaled
Ottawa, K1H 8L1 (CA)**
• **MOSQUERA, Lucy
Ottawa, K1P 5J6 (CA)**

(74) Representative: **Cobbold, Alistair John et al
Kilburn & Strode LLP
Lacon London
84 Theobalds Road
London WC1X 8NL (GB)**

(54) **SYSTEMS AND METHOD FOR EVALUATING IDENTITY DISCLOSURE RISKS IN SYNTHETIC PERSONAL DATA**

(57)    Although synthetic data synthesized from real sample data may not have a direct matching between synthetic data and individuals, there may still be a risk with identity disclosure. The identity disclosure risks associated with fully synthetic data may be assessed.

EP 3 901 791 A1

**Description**

**TECHNICAL FIELD**

The current disclosure relates to evaluating risks of identity disclosure in data, and in particular in synthetic data.

**BACKGROUND**

Access to data for AI and machine learning (AIML) projects has been problematic in practice. The Government Accountability Office and the McKinsey Global Institute both note that accessing data for building and testing AIML models is a challenge for their adoption more broadly. A Deloitte analysis concluded that data access issues are ranked in the top three challenges faced by companies when implementing AI.

A key obstacle to data access has been analyst concerns about privacy and meeting growing privacy obligations. A recent survey by O'Reilly highlighted the privacy concerns of companies adopting machine-learning models, with more than half of companies experienced with AIML checking for privacy issues. Specific to healthcare data, a recent NAM/GAO report highlights privacy as presenting a data access barrier for the application of AI in healthcare.

At the same time, the public is getting uneasy about how their data is used and shared, and regulatory scrutiny of secondary uses and disclosures of data is growing.

Different approaches have been proposed to facilitate the use and disclosure of health data for secondary purposes while significantly reducing obligations under current privacy statutes. Synthetic data generation is one such approach. Data synthesis has been highlighted as a key privacy enhancing technology to enable data access.

Previous identity disclosure assessment models for synthetic data that have been used in the literature were formulated under an assumption of partially synthetic data. Partially synthetic data permit direct matching of synthetic records with real people, but that assumption cannot be made with *fully* synthetic data whereby there is no direct mapping between a synthetic record and a real individual for all records. Further, previous identity disclosure assessment models did not consider that an adversary may attempt to identify individuals using all possible generalizations of variables (i.e., the previous attack models did not consider all possible generalizations that an adversary may try) and matching on a subset of variables which can in practice substantially increase the identification risk. Previous assessment models also failed to consider that an attack can be performed by finding a synthetic record that matches a target individual *or* by matching a synthetic dataset with a registry.

It would be desirable to have a new, improved and/or additional way to evaluate risks of identity disclosure for synthetic data.

**SUMMARY**

In accordance with the present disclosure, there is provided a method of determining an identity disclosure risk of synthetic sample data comprising: receiving a set of real sample records each of the real sample records associated with a respective individual in a population; receiving a set of synthetic sample records; determining if there is a match between synthetic records and real sample records; for real sample records determined to match synthetic records, determining probabilities of matching the matched real sample records to individuals; and determining an identity disclosure risk for the synthetic sample data based on the probability of matching the matched real sample records to individuals.

In a further embodiment of the method, determining a probability of matching the matched real sample records to individuals comprises: determining probabilities of matching individuals in the population to real sample records; and determining probabilities of matching real sample records to individuals in the population.

In a further embodiment of the method, the probability of matching a matched real sample record to an individual is the maximum of the probability of matching individuals in the population to the real sample record and the probability of matching the real sample record to individuals in the population.

In a further embodiment of the method, the probability of matching a matched real sample record to an individual is the probability of at least one of matching individuals in the population to the real sample record and matching the real sample record to individuals in the population.

In a further embodiment of the method, the identity disclosure risk for the synthetic sample is determined according to:

$$max\left(\frac{1}{N}\sum_{s=1}^{n}\frac{1}{f_s}\times I_s\times R_s, \frac{1}{n}\sum_{s=1}^{n}\frac{1}{F_s}\times I_s\times R_s\right).$$

In a further embodiment of the method, the identity disclosure risk for the synthetic sample is determined according to one of:
$$\lambda_{mid} \times max\left(\frac{1}{N}\sum_{s=1}^{n}\frac{1}{f_s} \times I_s \times R_s, \frac{1}{N}\sum_{s=1}^{n}\frac{1}{F_s} \times I_s \times R_s\right);$$
and

$$max\left(\frac{1}{N}\sum_{s=1}^{n}\frac{1}{f_s} \times \lambda_{mid} \times I_s \times R_s, \frac{1}{N}\sum_{s=1}^{n}\frac{1}{F_s} \times \lambda_{mid} \times I_s \times R_s\right);$$
where $\lambda_{mid}$ adjusts a probability of a correct match assuming perfect information by an attacker and is based on a verification rate of matches and an error rate of data.

In a further embodiment of the method, determining a match between synthetic records and real sample records uses hierarchical or other form of generalization of quasi-identifier variables.

In a further embodiment of the method, the determining a match between synthetic records and real sample records uses a generalization lattice, wherein after computing a match at a node in the generalization lattice, unmatched records of the real sample are removed from further matching.

In a further embodiment of the method, determining a match between synthetic records and real sample records uses a subset lattice for matching on a subset of quasi-identifier values.

In a further embodiment of the method, the method further comprises determining if new information is learned by matching the matched real sample records to individuals.

In a further embodiment of the method, the identity disclosure risk is further based on the determination of if new information is learned.

In accordance with the present disclosure there is further provided a method of determining matches between records in two datasets, the method comprising: generating generalization lattice of quasi-identifier variables used for matching records in the first dataset to records in the second dataset, wherein each node of the generalization lattice uses a generalization of at least one of the quasi-identifier variables; processing each node of the generalization lattice to determine if any of the records in the first dataset match records in the second dataset using the generalizations of the lattice node for the quasi-identifier variables; after processing each node, removing from further node processing any records in the second dataset that were not matched, wherein the lattice nodes are processed from a broadest generalization to a narrowest generalization.

In a further embodiment of the method, determining matches between records in two datasets further comprises: using a subset lattice wherein each node comprises respective subsets of quasi identifier variables.

In a further embodiment of the method, each node in the subset lattice is processed using a respective generalization lattice using the subset of quasi-identifiers of the node as the quasi-identifiers of the generalization lattice.

In accordance with the present disclosure there is further provided a non-transitory computer readable media storing instructions which when executed by a processor perform any of the methods as described above.

**BRIEF DESCRIPTION OF THE DRAWINGS**

Further features and advantages of the present disclosure will become apparent from the following detailed description taken in combination with the appended drawings, in which:

Fig. 1 depicts possible identity disclosure risks associated with synthetic data;

Fig. 2 depicts components of a system for evaluating identity disclosure risks in synthetic data;

Fig. 3 depicts a method of determining a meaning information disclosure risk;

Fig. 4 depicts a method of evaluating identity disclosure risks in synthetic data;

Fig. 5 depicts a further method of evaluating identity disclosure risks in synthetic data;

Fig. 6 depicts a further method of providing synthetic data having an acceptable identity disclosure risk;

Fig. 7 depicts hierarchies for generalization of variable values;

Fig. 8 depicts a generalization lattice for use in evaluating identity disclosure risks in synthetic data; and

Fig. 9 depicts a generalization lattice of subset- variable matching for use in evaluating identity disclosure risks in synthetic data.

**DETAILED DESCRIPTION**

Conceptually, the generation of synthetic data comprises first creating a model of the original data. This model captures the distributions and structure, such as correlations and interactions among the variables. The synthetic data is then sampled or generated from the model, which may be referred to as a synthesizer. A high utility synthetic dataset would have similar statistical properties as the original or real dataset.

While model-based methods for data synthesis were introduced in the early 90's, they were based on techniques borrowed from imputation (estimating missing values in data). Since then, there have been significant advances in synthesis methods, with more promising ones not requiring the specification of a model a priori, such as decision tree based approaches, and deep learning methods, such as Variational Auto Encoders and Generative Adversarial Networks (GANs).

Regardless of the techniques used to generate the synthetic data, data synthesis must balance data utility with data privacy. If the synthesizer is overfit to the original data, for example, then the synthetic data will be very similar to the original data making it easier to match synthetic records to individuals. Specifically, there may be a privacy concern with identity disclosure whereby a synthetic record could be correctly matched to a real person.

Some researchers have argued that fully synthetic data does not have an identity disclosure risk, for example, because there is no unique mapping between the records in the synthetic data with the records in the original data, with some researchers claiming that "identification of units and their sensitive data from synthetic samples is nearly impossible". Other researchers have noted that "it is widely understood that thinking of risk within synthetic data in terms of re-identification, which is how many other SDC [Statistical Disclosure Control] methods approach disclosure risk, is not meaningful"

The assumption that synthetic data does not have an identity disclosure risk is not necessarily correct. If the synthesizer is overfit then it is quite easy to generate synthetic datasets that replicate many of the original records, and would therefore have a high identity disclosure risk. Typically, original (untransformed) health or financial data will have an elevated risk of identity disclosure, and so an overfit synthesizer could generate synthetic data that could also have an elevated risk of identity disclosure. This may be the case because many datasets, particularly in the health field, have a high proportion of population unique records on the original variables, which makes the identity disclosure risk almost certain. Although the identity disclosure assessment techniques described herein are developed for use with fully synthetic data, they can also be applied to partially synthetic data.

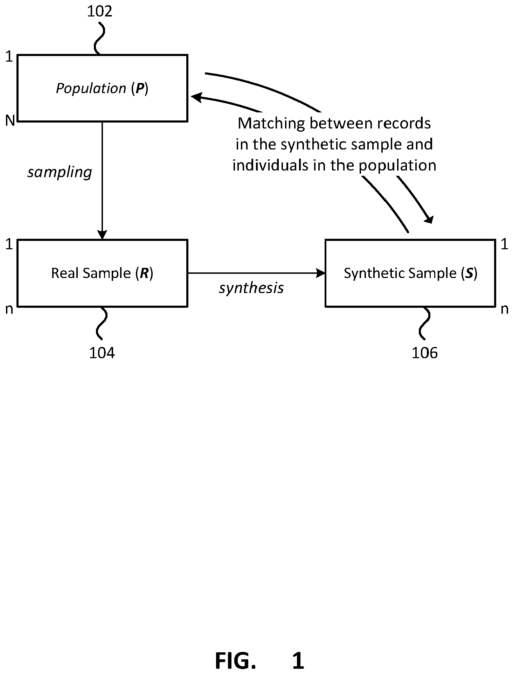

Fig. 1 depicts possible identity disclosure risks associated with synthetic data. As depicted in Fig. 1, an original population of data records (1..N) 102 may be sampled to generate real sample records (1..n) 104 which in turn are used to create synthetic sample records (1..n) 106. Although the synthetic records 106 are synthetically generated and as such may not have an exact 1:1 correspondence with the real samples, they may have enough information to permit a matching between an individual in the population with a synthetic record. As depicted in Fig. 1, there is a risk associated with matching a target individual to a synthetic record (i.e. population to synthetic sample data) and with matching a synthetic dataset with a registry (i.e. synthetic sample data to population). The systems and methods described further below may be used in evaluating both risks in determining an identity disclosure risk. In addition to evaluating the identity disclosure risk, which provides an indication of the likelihood or possibility of matching a real individual with a synthetic record, the systems and methods may also evaluate the amount of new, and correct information learned if a synthetic record is matched to an individual. That is, even if a synthetic record were matched to a real individual, there may be no disclosure risk if the only new information learned by an adversary from the synthetic record is inaccurate.

As depicted in Fig. 1 there may be a real population characterized by the set P of size *N.* A real sample R exists and that is the set that a synthetic dataset S is to be created from. Without loss of generality, the real and synthetic samples are assumed to be the same size *n* although they need not be.

Part of the risk evaluation process is matching records between the real sample and the synthetic sample. Matching may be performed on quasi-identifiers, which are a subset of the variables and that are known by an adversary. For example, typically a date of birth is a quasi-identifier because it is information about individuals that is typically known or that is relatively easy for an adversary to find out, such as from voter registration lists or social media.

The set of records, whether in population *P ,* real sample *R ,* or synthetic sample *S,* that have the same values on the quasi-identifiers are called an equivalence class. An equivalence class value is the set of values of the quasi-identifiers in that equivalence class. For example, if records 1, 2, and 3 in a dataset all have values Gender=male and Age=50 on the Gender and Age quasi-identifiers, then the equivalence class is records 1,2, and 3, and the equivalence class values are {Male, 50}.

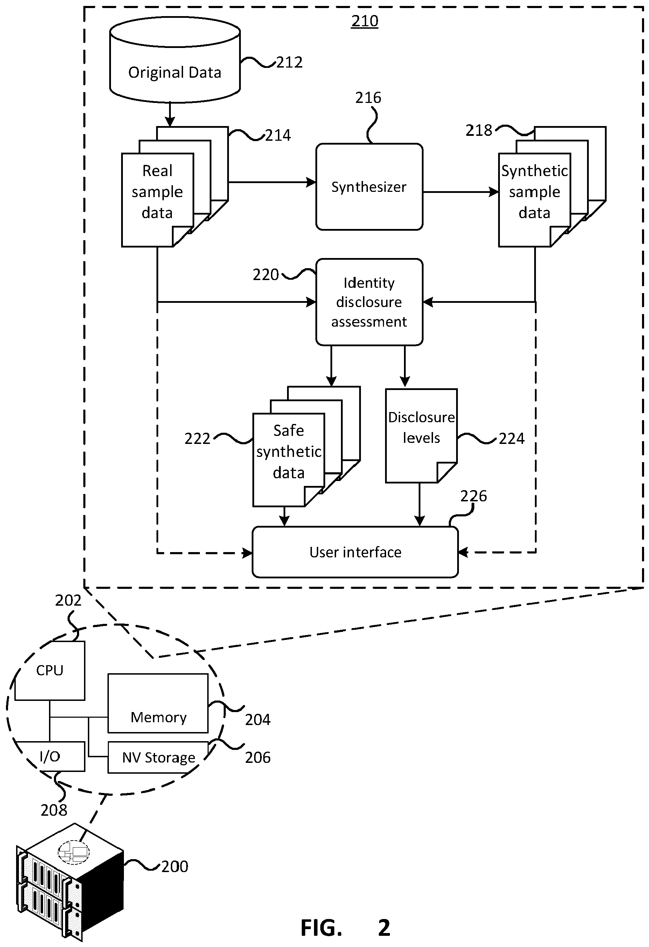

Fig. 2 depicts components of a system for evaluating identity disclosure risks in synthetic data. One or more computing devices 200, which are depicted as servers although other computing devices may be used, are depicted as implementing the one or more of the components of a system for generating synthetic data and evaluating the identity disclosure risk of the synthetic data. It will be appreciated that different components may be implemented on separate servers that are communicatively coupled to each other. The servers, or other computing devices used in implementing the components depicted, may include one or more central processing units (CPU) 202, one or more memory units 204, one or more non-volatile storage units 206 and one or more input/output interfaces (208). The one or more memory units 204 have stored thereon instructions, which when executed by the one or more processing units 202 of the one or more servers 200 configure the one or more servers to provide functionality components 210 for generating synthetic data and evaluating the identity disclosure risk of the synthetic data.

As depicted, the functionality components 210 may include a data source of original data 212, which may be for example the population P depicted in Fig. 1. In certain cases, the population may be conceptual as the only information available would be the real sample. A subset of the original data may be used as real sample data 214, which may be for example the real sample R in Fig. 1. The real sample data 214 may be used to create a synthesizer 216, which may generate the synthetic sample data, which may be for example the synthetic sample S in Fig. 1. As described in further detail below, identity disclosure assessment functionality 220 will use both the real sample data 214 and the synthetic sample data in order to determine an identity disclosure risk associated with the synthetic sample data. The identity disclosure assessment functionality 220 may output a safe synthetic data sample 222 and/or may output an indication of the identity disclosure risk levels 224 associated with synthetic sample data. A user interface component 226 may present the safe synthetic data and/or the identity disclosure risk levels may generate a user interface presenting the information to a user. The user interface component 226 may also include information from the real sample data 214 and the synthetic data 218 when generating the user interface. The user interface component 226 allows a user to utilize the identity disclosure assessment component to generate data sets. It will be appreciated that various application programming interfaces (APIs) may be provided in order to access the different components including the identity disclosure assessment component 220 and the synthesizer component 216 in order to incorporate the functionality into additional or alternative user interfaces and/or systems.

As described further below, the identity disclosure assessment may broadly determine the possibility of matching a synthetic record with a person. In certain applications simply identifying a synthetic record with a real person may be unacceptable. However, in other applications, simply matching a person to a synthetic record may be acceptable as long as no new meaningful information is learned by an attacker as a result of the matching. The identity disclosure assessment functionality described herein is able to evaluate both risks.

Table 1: Table describing notation

| $s$ | **An index to count records in the real sample** |
|---|---|
| $t$ | An index to count records in the synthetic sample |
| $N$ | The number of records in the true population |
| | |
| $f_s$ | The equivalence class group size in the sample for a particular real sample record $s$ |
| $F_s$ | The equivalence group size in the population that has the same quasi-identifier values as record $s$ in the real sample |
| $n$ | The number of records in the (real or synthetic) sample |
| $I_s$ | A binary indicator of whether record $s$ in the real sample matches a record in the synthetic sample |
| $R_s$ | A binary indicator of whether the adversary would learn something new if record $s$ in the real sample matches a record in the synthetic sample |
| $k$ | Number of quasi-identifiers |
| $\lambda$ | Adjustment to account for errors in matching and a verification rate that is not perfect |
| $L$ | The minimal percentage of sensitive variables that need to be similar between the real sample and synthetic sample to consider that an adversary has learned something new |

A concern with data sets is with meaningful identity disclosure. Meaningful identity disclosure is when an adversary is able to correctly assign an identity to a record in a dataset and by doing so learn something new about that individual. If an adversary is able to correctly assign an identity to a record but does not learn anything new, then, arguably, that is not a meaningful identity disclosure. Although a meaningful identity disclosure may occur if something new has been learned, it can be beneficial to determine if an attacker is able to assign an identity to a record in a dataset even if something new may not have been learned.

Accordingly, the identity disclosure assessment may apply two sequential tests on a synthetic sample to determine its identity disclosure risks. First, the extent to which synthetic sample records can be matched to real individuals in the population is determined, and where a match is made, the extent to which there is correct information gain by the adversary is determined. A record in the synthetic sample must pass both tests to be deemed to have a high risk of meaningful identity disclosure.

The probability of a successful match between someone in the population and a synthetic record will depend on the direction of the match. This is illustrated in Fig. 1. Matching between a synthetic sample record and someone in the population goes through the real sample, and can occur in two directions. One direction is from the population to the synthetic sample, and the second direction is from the synthetic sample to the population. The probability of a correct match will be different depending on the direction.

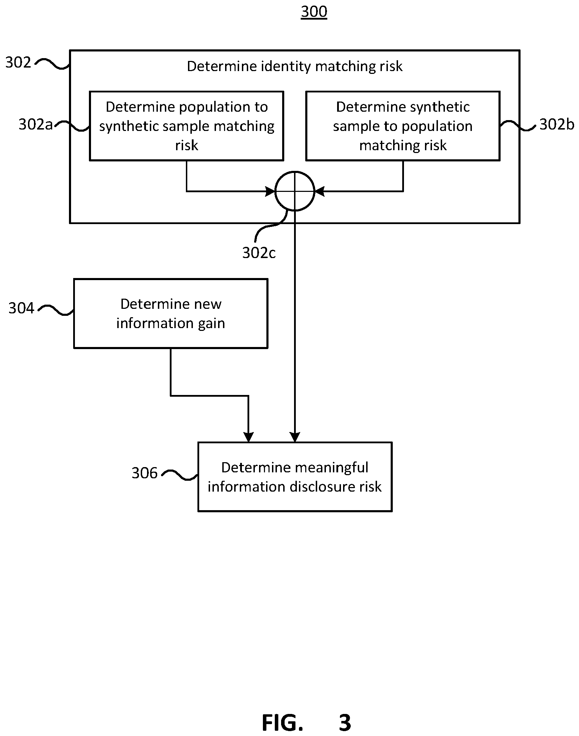

Fig. 3 depicts a method of evaluating identity disclosure risks in synthetic data. The method 300 determines an identity matching risk (302), which is the risk of matching records in the synthetic sample with individuals in the population. The identity matching risk 302 may be determined based on two matching risks and as such may comprise determining a population to synthetic sample matching risk (302a) and determining the synthetic sample to population matching risk (302b). The two determined risks are then combined (302), for example by taking the maximum of the two determined risks, to provide the identity matching risk. Alternatively, the identity matching risk may be provided as the probability that at least one of the attacks will occur. In addition to the identity matching risk, the new information gained from a successful matching is also determined (304), and the new information gained along with the identity matching risk used to determine the meaningful information disclosure risk (306). The meaningful information disclosure risk provides an indication of not only whether synthetic sample records may be matched to a real individual but also whether any new information is gained about the individual by making such a match.

It is possible to formulate an overall probability of identification for a synthetic record as follows:

$$pr\left(real\_match \middle| synthetic\_match\right) \times pr\left(synthetic\_match\right) \qquad (1)$$

This probability can be calculated in both directions of attack, namely the population to the synthetic sample and the synthetic sample to the population. The terms in equation (1) are defined as follows:

*synthetic_match* is the matching of a synthetic sample record to a real sample record on the quasi-identifiers. This by itself does not mean that a synthetic sample record can be identified, but it is a necessary step in matching; and

*real_match* is when a record in the real sample is matched to an identity of an individual in the population.

The first part for evaluating *pr(synthetic_match)* is to match a synthetic sample record with a real sample record. Consider the synthetic sample in Table 1 below with a single quasi-identifier, namely origin. An attacker desires to match the record with the *Hispanic* value with the real sample in Table 2. There are three matching records in the real sample. Without any further information, one of the three real sample records would be selected at random, and therefore the probability of selecting any of the records is $1/3$. However, there is no correct selection here since the sample is fully synthetic. For example, it is not possible to say that record #3 in the real sample is the correct record to match with and therefore the probability of a correct match is $1/3$. There is no 1:1 mapping between the fully synthetic sample records and the real sample records.

The key information here is that there was a match - it is a binary indicator. If there is a match between real sample record $s$ and a synthetic record, then the indicator $I_s$ is used, which takes on a value of one if there is at least one match, and zero otherwise.

| Origin | Income |
|---|---|
| Japanese | $120k |
| North African | $100k |
| European | $110k |
| Hispanic | $65k |

Table 2: Example synthetic sample with Origin being the quasi-identifier.

| ID | Origin | Income |
|----|----------|--------|
| 1 | European | $70k |
| 2 | Hispanic | $100k |
| 3 | Hispanic | $130k |
| 4 | Hispanic | $65k |

Table 3: Example real sample with Origin being the quasi-identifier.

The basic model for computing *pr(real_match|synthetic_match)* is described further below as well as how to extend the basic model to account for the matches since only those records that match between the real and synthetic samples can be associated with a person in the population.

It is possible to assess the probability that a record in the real sample can be identified by matching it with an individual in the population by an adversary. There are two directions of attack by an adversary. The first is when the adversary knows someone in the population (the target individual) and attempts to match that individual to a record in the real sample. This will be referred to as a population-to-sample attack (*A*). The second is when the adversary selects a record in the real sample and attempts to match it with records in the population. This will be referred to as a sample-to-population attack (*B*).

Under the assumption that an adversary will only attempt one of them, but it is not known which one, the overall probability of one of these attacks being successful may be given by the maximum of both:

$$\text{Max}(A, B) \tag{2}$$

Rather than taking the maximum between the two attacks, the overall risk probability may be given as the probability of at least one of the attacks be successful and may be given as:

$$1 - (1 - A)(1 - B) \tag{3}$$

The manner in which the population-to-sample risk has traditionally been measured is quite conservative, resulting in potentially inflated identity disclosure risk estimates. While conservatism may be acceptable from the perspective of protecting patient privacy, it also means that the extent of transformations that are needed in a dataset to ensure that it has acceptably low identity disclosure risk will be more extensive than necessary - resulting in a reduction in data utility. Low data utility affects the ability to perform meaningful health research, for example, on data that is deemed to have a low risk of identity disclosure. Therefore, by adjusting this conservatism, the process is better able to ensure that identity disclosure risks for patients are low and that the resultant data utility remains high for beneficial uses of data. The average population-to-sample match rate may be expressed in terms of individual records rather than equivalence classes as:

$$A = \frac{1}{N}\sum_{s=1}^{n}\frac{1}{f_s} \tag{4}$$

For a sample-to-population attack, an adversary would match records from the sample datasets to the represented population. The risk value for a sample-to-population attack is:

$$B = \frac{1}{n}\sum_{s=1}^{n}\frac{1}{F_s} \tag{5}$$

Accounting for whether a record in the real sample matches a record in the synthetic sample which is indicated by $I_s$, the risks for the population-to-sample can be extended to the risk for the population-to-synthetic sample according to:

$$A = \frac{1}{N}\sum_{s=1}^{n}\frac{1}{f_s} \times I_s \tag{6}$$

The risks for the sample-to-population can be extended to the risk for the synthetic sample-to-population according to:

$$B = \frac{1}{n}\sum_{s=1}^{n}\frac{1}{F_s} \times I_s \qquad (7)$$

The overall identity disclosure risk is given by:

$$max\left(\frac{1}{N}\sum_{s=1}^{n}\frac{1}{f_s} \times I_s, \frac{1}{n}\sum_{s=1}^{n}\frac{1}{F_s} \times I_s\right) \qquad (8)$$

The value of $\frac{1}{F_s}$ can be estimated using methods as described in K. El.Emam, Guide to the De-Identification of Personal Health Information, CRC Press (Auerbach), 2013 which is incorporated herein by reference in in its entirety for all purposes.

In practice there are two adjustments that can be made to equation (8) to take into account the reality of matching when attempting to identify records: verification and data errors.

A previous review of identification attempts found that when there is a suspected match between a record and a real individual, that suspected match could only be verified 23% of the time. This means that a large proportion of suspected matches turn out to be false positives when the adversary attempts to verify them.

Additionally, real data typically has errors in it and therefore the accuracy of the matching based on adversary knowledge will be reduced. Known data error rates not specific to health data (e.g., voter registrations, surveys, and data brokers) can be relatively large. For health data, the error rates have tended to be lower with a weighted mean of 4.26%. Therefore, erring on the conservative side, the probability of at least one variable having an error in it is given by $1-(1-0.0426)^k$, where $k$ is the number of quasi-identifiers. If it is assumed that the adversary has perfect information and only the data will have an error in it, then the probability of a correct match is $(1-0.0426)^k$. It is noted that the application to health data is only one example and the model can be applied to other types of data. The weighted mean of 4.26% may differ in other domains.

Therefore, equation (8) can be adjusted with the λ parameter:

$$\lambda \times max\left(\frac{1}{N}\sum_{s=1}^{n}\frac{1}{f_s} \times I_s, \frac{1}{n}\sum_{s=1}^{n}\frac{1}{F_s} \times I_s\right) \qquad (9)$$

Where:

$$\lambda = 0.23 \times (1 - 0.0426)^k \qquad (10)$$

Equation (9) above applies the adjustment parameter λ after calculating the maximum of the two matching probabilities. However, the λ parameter may be calculated for each iteration of calculating the risk for the population-to-synthetic sample and the risk for the synthetic sample-to-population. In such an embodiment, equation (8) can be adjusted with the λ parameter according to:

$$max\left(\frac{1}{N}\sum_{s=1}^{n}\frac{1}{f_s} \times \lambda \times I_s, \frac{1}{n}\sum_{s=1}^{n}\frac{1}{F_s} \times \lambda \times I_s\right) \qquad (11)$$

The above assumes that verification rates and error rates are independent, however they are unlikely to be so. Specifically, when there are data errors that would make the ability to verify less likely, which makes these two entities correlated. The correlation can be captured.

The verification rate and error rate can be represented as triangular distributions, which is a suggested way to model phenomena for risk assessment where the real distribution is not precisely known. The minimum and maximum values can be taken from the literature. The correlation may be assumed to be medium according to Cohen's guidelines for the interpretation of effect size, although other assumptions can be made. We can then sample from these two distributions inducing a medium correlation. The actual sampled values can be used in equation (10) instead of the fixed values. $\lambda_s$ provides the adjustment parameter when using the sampled values. Regardless of whether the adjustment parameter is calculated using the fixed values (i.e. $\lambda$) or the sampled values (i.e. $\lambda_s$), it is possible to use the mean value of either $\lambda$ or $\lambda_s$, namely $\overline{\lambda}$ or $\overline{\lambda_s}$ respectively, instead.

Rather than using $\lambda$ or $\lambda_s$ (or $\overline{\lambda}$ or $\overline{\lambda_s}$) directly, the midpoint between $\lambda$ or $\lambda_s$ and a maximum value of 1 may be used. The actual risk value can be selected to be the midpoint of that range:

$$\lambda_{mid} = \frac{1+\lambda}{2} \tag{12}$$

Similarly, if the sampled values are used rather than the fixed values, the midpoint value may be:

$$\lambda_{mid} = \frac{1+\lambda_s}{2} \tag{13}$$

Regardless of whether the midpoint value is determined according to equation (12) or (13) above, the identity disclosure risk may be calculated as:

$$\lambda_{mid} \times max\left(\frac{1}{N}\sum_{s=1}^{n}\frac{1}{f_s} \times I_s, \frac{1}{n}\sum_{s=1}^{n}\frac{1}{F_s} \times I_s\right) \tag{14}$$

Alternatively, $\lambda_{mid}$ may be used in equation (11), and the identity disclosure risk may be calculated as:

$$max\left(\frac{1}{N}\sum_{s=1}^{n}\frac{1}{f_s} \times \lambda_{mid} \times I_s, \frac{1}{n}\sum_{s=1}^{n}\frac{1}{F_s} \times \lambda_{mid} \times I_s\right) \tag{15}$$

Equation (14), or (15), can provide the overall identity disclosure risk for matching a record in a synthetic sample with an individual. The identity disclosure risk can be extended to account for whether or not an attacker that has matched a synthetic record with an individual will learn new meaningful data from the match.

Equation (14) is extended to determine if the adversary would learn something new from a match. Letting $R_s$ be a binary indicator of whether the adversary could learn something new, equation (14) becomes:

$$\lambda_{mid} \times max\left(\frac{1}{N}\sum_{s=1}^{n}\frac{1}{f_s} \times I_s \times R_s, \frac{1}{n}\sum_{s=1}^{n}\frac{1}{F_s} \times I_s \times R_s\right) \tag{16}$$

Similarly, equation (15) may be extended according to:

$$max\left(\frac{1}{N}\sum_{s=1}^{n}\frac{1}{f_s} \times \lambda_{mid} \times I_s \times R_s, \frac{1}{n}\sum_{s=1}^{n}\frac{1}{F_s} \times \lambda_{mid} \times I_s \times R_s\right) \tag{17}$$

In practice $I_s$ may be calculated first for records and if that is a zero then there is no point in computing the remaining terms in the max function parameters: it is only necessary to consider those records that have a match between the real and synthetic samples since the "learning something new" test would not be applicable where there is no match.

Learning something new in the context of synthetic data can be expressed as a function of the non-quasi-identifiers and the quasi-identifiers that were not involved in the match between the synthetic sample and real sample. These variables will be called the *sensitive* variables since the assumption that learning something new on these sensitive variables would potentially be harmful to the patients. Also note that for the analysis it is assumed that the sensitive variable is at the same level of granularity as in the original real data since that is the information that the adversary will have after a match.

The test of whether an adversary learns something new is defined in terms of two criteria:

1. is the individual's real information different than other individuals in the real sample (i.e., to what extent is that individual an outlier in the real sample); and

2. to what extent is the synthetic sample value similar to the real sample value.

Both of these conditions would be tested for every sensitive variable. The relationship between a real observation to the rest of the data in the real sample and to the synthetic observation, and how that can be used to determine the likelihood of meaningful identity disclosure is depicted in Table 3, which only applies to records that match between the synthetic and real data, and hence have passed the first test for what is defined as meaningful identity disclosure.

| | | Similarity in Real Sample | |
|---|---|---|---|
| | | **Individual is Similar to Others** | **Individual is an Outlier** |
| **Similarity Between Real & Synthetic Samples** | **Individual's Synthetic Information Similar to Real Information** | Low Meaningful Identity Disclosure Risk | High Meaningful Identity Disclosure Risk |
| | **Individual's Synthetic Information Different from Real Information** | Low Meaningful Identity Disclosure Risk | Low Meaningful Identity Disclosure Risk |

Table 3: The relationship between a real observation to the rest of the data in the real sample and to the synthetic observation, and how that can be used to determine the likelihood of meaningful identity disclosure.

If, for example the sensitive variable being looked at is the cost of a procedure. Consider the following scenarios:

- If the real information about an individual is very similar to other individuals (e.g., the value is the same as the mean), then the information gain from an identification would be low (note that there is still some information gain, but it would be lower than the other scenarios). However, if the information about an individual is quite different, say the cost of the procedure is three times higher than the mean, then the information gain could be relatively high because that value is unusual.

- If the synthetic cost is quite similar to the real cost then the information gain is higher still. However, if the synthetic cost is quite different from the real cost then very little would be learned by the adversary or what will be learned will be incorrect, and therefore the correct information gain would be low.

This set of scenarios is summarized in Table 4 above. Only one quadrant would then represent a high and correct information gain and the objective of the risk assessment is to determine whether a matched individual is in that quadrant for at least $L\%$ of its sensitive variables. A reasonable value of $L$ would need to be specified for a particular analysis.

A model is provided below to assess what the adversary would learn from a sensitive variable. The difference between the real and synthetic values may then be considered. If the adversary learns something new for at least $L\%$ of the sensitive variable, then set $R_s = 1$ otherwise it is zero.

Development of the model of learning something new starts off with nominal/binary variables and then the model is extended to continuous variables. Let $X_s$ be the sensitive variable for real record $s$ under consideration, and let $J$ be the set of different values that $X_s$ can take in the real sample. Assume the matching record has value $X_s = j$ where $j \in J$, and that $p_j$ is the proportion of records in the whole real dataset that have the same $j$ value.

It is possible to then determine the distance that the $X_s$ value has from the rest of the real sample data as follows:

$$d_j = 1 - p_j \tag{18}$$

Let the matching record on the sensitive variable in the synthetic sample be denoted by $Y_t = z$, where $z \in Z$ and $Z$ is the set of possible values that $Y_t$ can take in the synthetic sample. The values of any two records that match from the real sample and the synthetic sample can be compared. The measure of how similar the real value is to the rest of the distribution when it matches is therefore given by $d_j \times I(X_s = Y_t)$, where $I(\ )$ is the indicator function.

In order to determine if the value indicates that the adversary learns something new about the patient, a conservative threshold is set such that if the similarity is larger than one standard deviation the adversary is considered to learn something new, assuming that taking on value $j$ follows a Bernoulli distribution. The inequality for nominal and binary variables that must be met to declare that an adversary will learn something new from a matched sensitive variable is:

$$d_j \times I\left(X_s = Y_t\right) > \sqrt{p_j\left(1 - p_j\right)} \qquad (19)$$

The inequality compares the weighted value with the standard deviation of the proportion $p_j$.

The above has described determining if an adversary learns new information using nominal/binary sensitive variables. The determination may be extended to continuous sensitive variables. Continuous sensitive variables were discretized using univariate k-means clustering, with optimal cluster sizes chosen by the majority rule. Again let $X$ be the sensitive variable under consideration, and the value on that variable for the real record under consideration to be $X_s$. The size of the cluster in the real sample where the value of the sensitive variable that belongs to the matched real record being examined can be defined as $C_s$. For example, if the sensitive variable is the cost of a procedure and it is $150. If that specific value is in a cluster of size 5, then $C_s = 5$. The proportion of all patients that are in this cluster compared to all patients in the real sample is given by $p_s$.

In the same manner as for nominal and binary variables, the similarity is defined as:

$$d_s = p_s \qquad (20)$$

Let $Y_t$ be the synthetic value on the continuous sensitive variable that matched with real record $s$. The weighted absolute difference expresses how much information the adversary has learned $d_s \times |X_s - Y_t|$.

It is desirable to determine if this value signifies learning too much. This value is compared to the median absolute deviation (MAD) over the $X$ variable. The MAD is a robust measure of variation. We define the inequality:

$$d_s \times \left|X_s - Y_t\right| \le 1.48 \times MAD \qquad (21)$$

When this inequality is met then the weighted difference between the real and synthetic values on the sensitive variable for a particular patient indicates that the adversary will indeed learn something new.

The 1.48 value makes the MAD equivalent to one standard deviation for Gaussian distributions. Of course, the multiplier for MAD can be adjusted since the choice of a single standard deviation equivalent was a subjective decision.

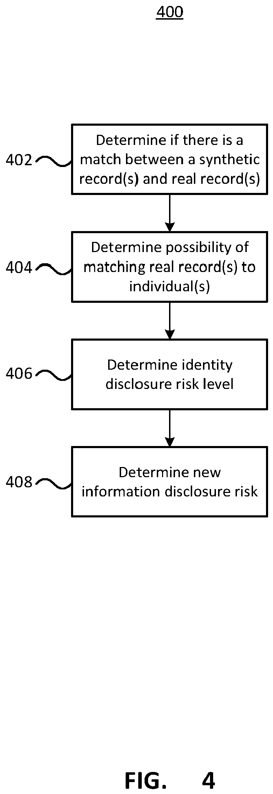

Fig. 4 depicts a further method of evaluating identity disclosure risks in synthetic data. The method 400 determines if there is a match between synthetic record(s) and real record(s) (402). As described above, whether there is a match between synthetic and real records is determined as a binary variable $I$. After determining if there is a match between synthetic and real records, the possibility of matching real record(s) to individual(s) is determined (402). As described above, the possibility of the matching may be determined according to equation (8). The determination of whether or not a real record may be matched to an individual may be done before or after determining if there is a match between synthetic and real records, however, if it is determined after, only those real records that are determined to match synthetic records need to be considered further. The identity disclosure risk level may be determined (406), for example according to equation (14), or (15). The identity disclosure risk level provides an indication of the risk of matching a synthetic record with an individual. The new information disclosure risk, which indicates the risk that new information may be learned when a synthetic record is matched to an individual, may be determined (408) according to equation (16), or (17).

The above has described determining the identity disclosure risk and/or new information disclosure risk for a data set as a whole. As described further below, the risks for individual records may be calculated.

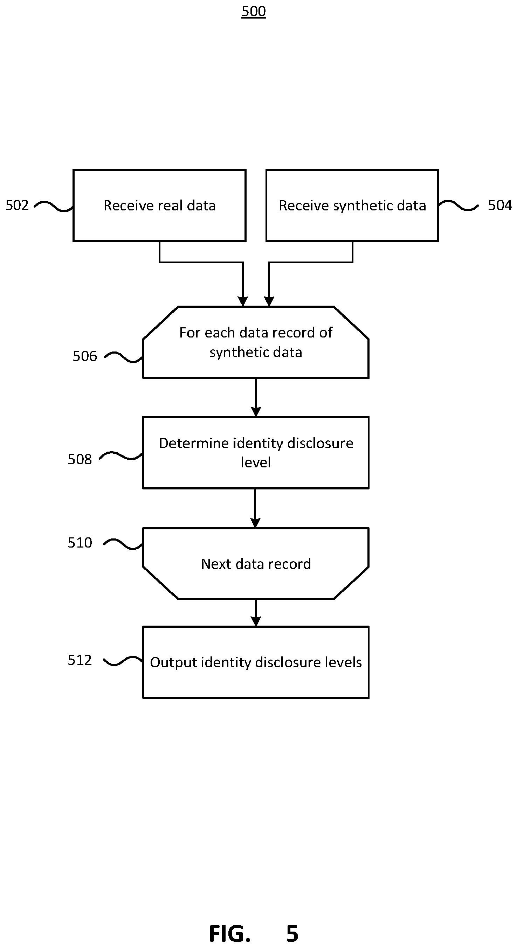

Fig. 5 depicts a further method of providing synthetic data having an acceptable identity disclosure risk. As depicted, the method 500 receives real sample data (502) and synthetic sample data (504). For each record of the synthetic sample data (506), the identity disclosure level may be determined (508), and then the next record (510) processed (510). In determining the identity disclosure risk of a record, it may be determined whether the synthetic record being considered matches a real record or records. If there is a match in the real sample records for the synthetic sample record, the possibility of matching the real record with an individual may then be determined, for example according to max($A, B$), although other methods of determining the possibility can be used such as the probability that at least one of the attacks will be successful. Once the disclosure risk levels have been determined for all records, the identity disclosure risk levels may be output (512). The disclosure risk levels that are output may be used in various ways, including for example determining an overall identity disclosure risk of the entire sample, identifying high risk records, etc.

As described above, identity disclosure risks may be determined for the sample as a whole and/or for individual records. The above has assumed that the synthesis of the synthetic data is separate from the evaluation of the samples. As described further below, it is possible to incorporate the sample synthesis with the identity disclosure risk analysis.

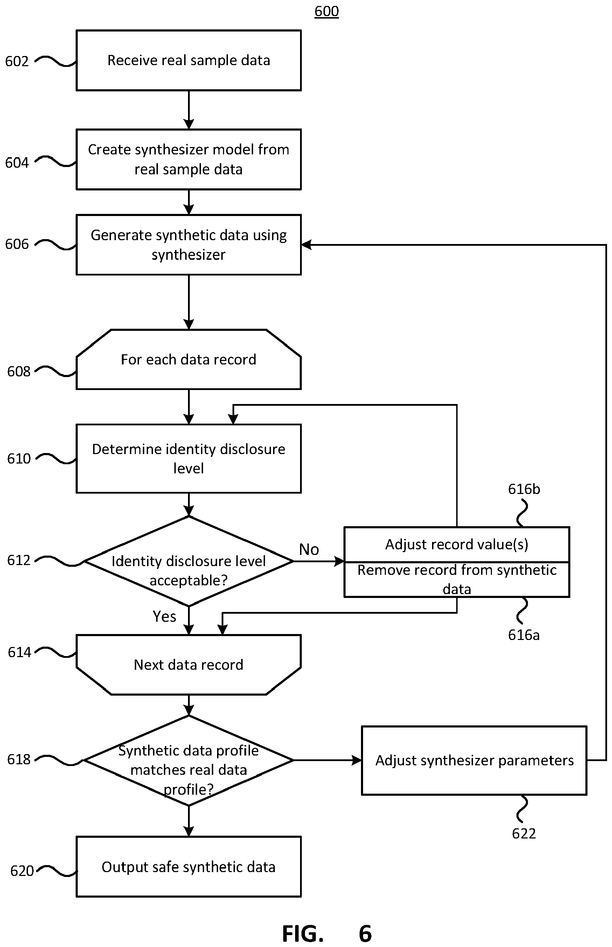

Fig. 6 depicts a further method of providing synthetic data having an acceptable identity disclosure risk. The method 600 receives real sample data (602) and creates a synthesizer model from the real sample data (604). The synthesizer model is then used to generate synthetic sample data (606) and each of the records of the synthetic sample data (608) may then be processed. The identity disclosure risk of the record may be determined (610) and if the identity disclosure risk is acceptable (Yes at 612) the next record (614) may be processed. If the identity disclosure risk of the record is not acceptable (No at 612), one or more mitigating actions may be taken. For example, the record may be removed from the synthetic data 616a and the next record processed (614). Alternatively, one or more values of the record may be adjusted and the identity disclosure risk of the adjusted record reassessed (610). Once all of the records of the synthetic sample records have been processed, the synthetic sample profile may be compared to the real data profile to determine if they match, possibly within a similarity threshold value. The synthetic sample profile and real sample profile may characterize the samples, such as the distribution of values, relationship of variables, etc. The closer the synthetic sample profile is to the real sample data profile the more useful the synthetic data may be. If the profiles do match, possibly within a similarity threshold, the synthetic data may be output. The output synthetic data may be used in various ways including for example in AIML applications. If the profiles do not match, possibly within a similarity threshold, the parameters of the synthesizer used to generate the synthetic sample may be adjusted (622) and new synthetic data generated (606). Alternatively, rather than adjusting parameters of an existing synthesizer, a new synthesizer could be created and new synthetic sample records generated using the new synthesizer.

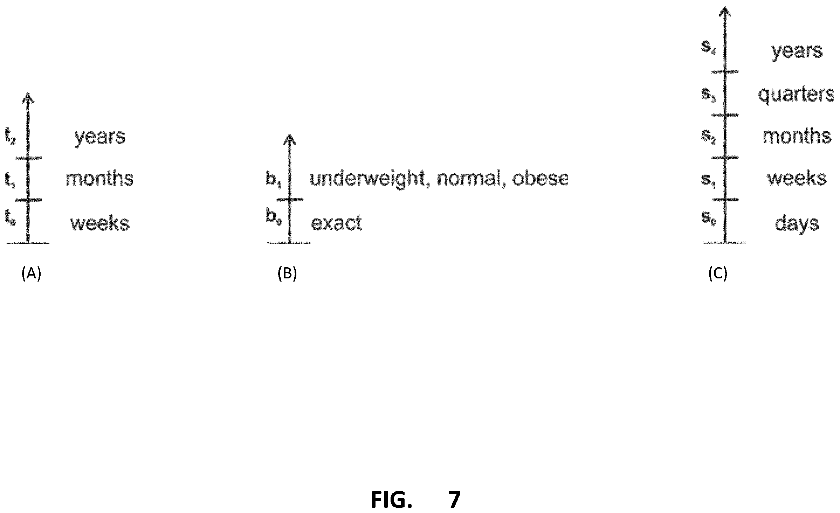

The above has considered that an adversary will attempt to match records using original values, however this need not be the case. An adversary may generalize the values and match on those. Therefore, it is necessary to evaluate the risks for matching on generalized values as well. Generalizations can be expressed in terms of hierarchies as illustrated in Fig. 7, which depicts various hierarchies for generalization of variable values.

Fig. 7 depicts examples of three generalization hierarchies for (A) time on the study for say a clinical trial, (b) BMI, and (c) survival. As one moves up the hierarchy the level of granularity decreases, and this level is indicated by the notation on the left.

The more generalizations that are applied to the synthetic and real samples, the greater the chance of a match between the synthetic sample and the real sample. For example, if the synthetic sample is matched with the real sample on the exact BMI then it may not match any patients, but if the samples are generalized to the $b_1$ categories in panel (b) in Fig. 7, then the match rate does increase. At the same time, as the data are generalized, the risk of identification of the real data will likely decrease.

Determining the risk associated with generalization of variables can be computationally expensive as the risk associated with each generalization needs to be determined. As described further below, the risk may be evaluated using a generalization lattice that allows the risk of broader generalizations to be used in determining the risks of narrower generalizations.

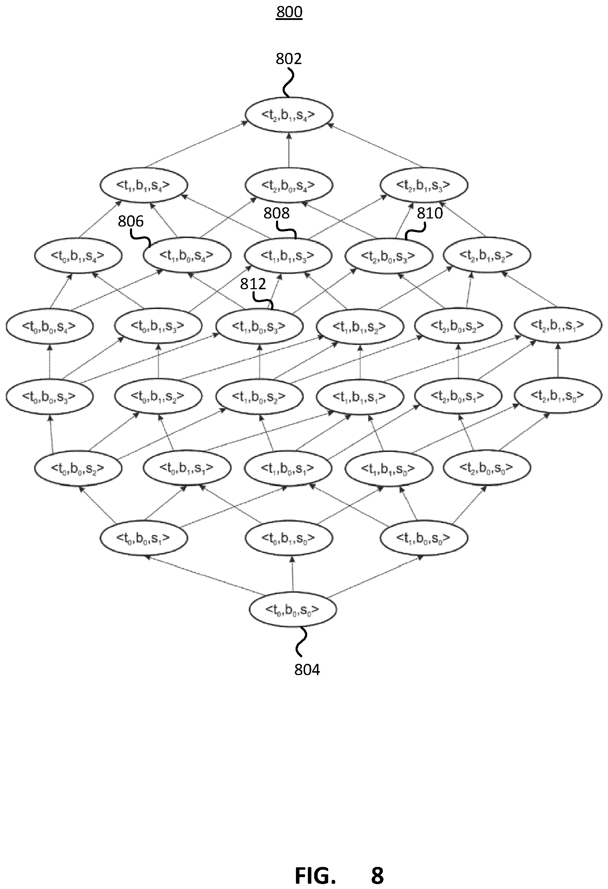

It is possible to represent all possible generalizations on the quasi-identifiers as a *generalization lattice,* depicted in FIG. 8. In a generalization lattice the least generalized version of the quasi-identifiers is at the bottom (i.e., the lowest granularity which is the original data), and the top of the lattice is the most generalized version of the quasi-identifiers. Each node represents a further generalization of a single quasi-identifier compared to the nodes below it in the lattice. The lattice represents all possible generalizations that an adversary can attempt on the synthetic data to identify it.

Fig. 8 depicts a generalization lattice for use in evaluating identity disclosure risks in synthetic data with generalization of variables. As the nodes move up the lattice the likelihood of a match between the real sample and synthetic sample will by definition stay the same or *increase.* The top node 802 represents the broadest generalizations of the quasi-identifier variables while the bottom node 804 represents the most specific quasi-identifier values. In addition, as the lattice is traversed upwards, the risk of identification (matching a real sample record with a real person in the population or vice versa) will by definition stay the same or decrease.

As one navigates the lattice, it is possible to test the following inequality at each node representing particular generalization levels of the quasi-identifiers:

$$\lambda_{mid} \times \max\left( \frac{1}{N}\sum_{s=1}^{n}\left(\frac{1}{f_s}\times I_s \times R_s\right), \frac{1}{n}\sum_{s=1}^{n}\left(\frac{1}{F_s}\times I_s \times R_s\right)\right) \leq \tau$$

(22)

where $\tau$ is some threshold of acceptable risk. When the inequality is tested on every node in the lattice, there are two possible outcomes:

1. The inequality is satisfied: the risk of identity disclosure is considered low and therefore no further action is needed.

2. The inequality is not satisfied, and therefore it is desirable to assess whether something new is learned or not - this is the second test for meaningful identity disclosure.

Alternatively, the following inequality may be tested at each node:

$$max\left(\frac{1}{N}\sum_{s=1}^{n}\frac{1}{f_s}\times \lambda_{mid}\times I_s \times R_s, \frac{1}{n}\sum_{s=1}^{n}\frac{1}{F_s}\times \lambda_{mid}\times I_s \times R_s\right) \leq \tau$$

(23)

To navigate the lattice for the inequality evaluation, the top node is processed first and subsequently lower nodes are processed. As a node passes the inequality test, the real records that were not matched are removed from the real sample since if they do not match in a general case they will also not match in a more specific case. Accordingly the dataset size that is being processed to perform the calculation in equation (22), or (23), will gradually decrease as the lattice is processed from top to bottom, speeding up the computations.

To take advantage of that pattern to reduce the amount of computation, every node need only consider the records that have matched in nodes higher up in the lattice hierarchy along the defined generalization paths. For example, nodes 806, 808, 810 are computed before node 812. In this case, the intersection of real sample patients that matched (i.e., $I_s$=1 ) in nodes $<t_1,b_0,S_4>$, $<t_1,b_1,S_3>$ and $<t_2,b_0,S_3>$ will be used to perform the computations in $<t_1,b_0,S_3>$.

The above assumes that all quasi-identifiers are used in matching. Similar to the generalization of the quasi-identifier values, matching may be done on a subset of quasi identifiers.

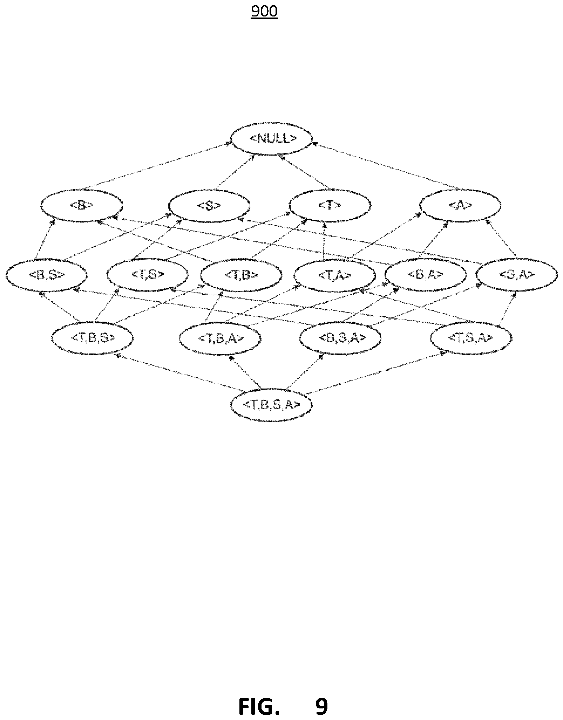

Fig. 9 depicts a generalization lattice of subset- variable matching for use in evaluating identity disclosure risks in synthetic data. If the number of quasi-identifiers is denoted by $k$, an adversary may also try to match on fewer than k variables. By definition, the fewer the number of variables the more likely it is to match synthetic records with real records. At the same time, the identification risk will decrease the fewer quasi-identifiers are considered. The different combinations of quasi-identifiers can be represented as a *subsets lattice* as illustrated in Fig. 9. At the bottom of the lattice are all of the quasi-identifiers, and as the lattice is traversed upwards the number of variables decreases. Each node of Fig. 9 can be computed as a generalization lattice as described with reference to Fig. 8.

In practice, the lattice navigation would start with the variable subsets lattice, and for every node there perform the computations on the generalization lattice for that subset of quasi-identifiers. Also, computations on the subset lattice should start from the top moving down. Further, for every node in the generalization lattice, unmatched record elimination can be performed on the nodes above it also for the nodes above it in the subsets lattice. For example, node $<t_0,b_0,s_2>$ would eliminate unmatched records in node $<t_0,b_0>,<t_0,s_2>,<b_0,s_2>,<t_0>,<b_0>,<s_2>$ and those above them in their respective generalization lattice. This can reduce the number of records that need to be considered and so reduce the computation.

The lattice may be used in matching records as well as determining overall risk of the synthetic data. The lattice may be used to compute the risk for each node. The risk calculations across the lattice nodes may then be aggregated to determine what the overall risk of the synthetic data is. One form of aggregation is to take the maximum risk calculated across all of the nodes, however other aggregations can be used.

The above has described systems and methods that may be useful in determining an identity disclosure risk of fully synthetic sample data. Particular examples have been described with reference to health related data. It will be appreciated that, while identity disclosure risk evaluation may be important in the health field, the above also applies to evaluating disclosure risks in other domains.

Although certain components and steps have been described, it is contemplated that individually described components, as well as steps, may be combined together into fewer components or steps or the steps may be performed sequentially, non-sequentially or concurrently. Further, although described above as occurring in a particular order, one of ordinary skill in the art having regard to the current teachings will appreciate that the particular order of certain steps relative to other steps may be changed. Similarly, individual components or steps may be provided by a plurality of components or steps. One of ordinary skill in the art having regard to the current teachings will appreciate that the components and processes described herein may be provided by various combinations of software, firmware and/or hardware, other than the specific implementations described herein as illustrative examples.

The techniques of various embodiments may be implemented using software, hardware and/or a combination of software and hardware. Various embodiments are directed to apparatus, e.g. a node which may be used in a communications system or data storage system. Various embodiments are also directed to non-transitory machine, e.g., computer, readable medium, e.g., ROM, RAM, CDs, hard discs, etc., which include machine readable instructions for controlling a machine, e.g., processor to implement one, more or all of the steps of the described method or methods.

Some embodiments are directed to a computer program product comprising a computer-readable medium comprising code for causing a computer, or multiple computers, to implement various functions, steps, acts and/or operations, e.g. one or more or all of the steps described above. Depending on the embodiment, the computer program product can, and sometimes does, include different code for each step to be performed. Thus, the computer program product may, and sometimes does, include code for each individual step of a method, e.g., a method of operating a communications device, e.g., a wireless terminal or node. The code may be in the form of machine, e.g., computer, executable instructions stored on a computer-readable medium such as a RAM (Random Access Memory), ROM (Read Only Memory) or other type of storage device. In addition to being directed to a computer program product, some embodiments are directed to a processor configured to implement one or more of the various functions, steps, acts and/or operations of one or more methods described above. Accordingly, some embodiments are directed to a processor, e.g., CPU, configured to implement some or all of the steps of the method(s) described herein. The processor may be for use in, e.g., a communications device or other device described in the present application.

Numerous additional variations on the methods and apparatus of the various embodiments described above will be apparent to those skilled in the art in view of the above description. Such variations are to be considered within the scope.

## Claims

1. A computer-implemented method of determining an identity disclosure risk of synthetic sample data comprising:

   receiving a set of real sample records each of the real sample records associated with a respective individual in a population;
   receiving a set of synthetic sample records;
   determining if there is a match between synthetic records and real sample records;
   for real sample records determined to match synthetic records, determining probabilities of matching the matched real sample records to individuals; and
   determining an identity disclosure risk for the synthetic sample data based on the probability of matching the matched real sample records to individuals.

2. The computer-implemented method of claim 1, wherein determining a probability of matching the matched real sample records to individuals comprises:

   determining probabilities of matching individuals in the population to real sample records; and
   determining probabilities of matching real sample records to individuals in the population.

3. The computer-implemented method of claim 1, wherein the probability of matching a matched real sample record to an individual is the maximum of the probability of matching individuals in the population to the real sample record and the probability of matching the real sample record to individuals in the population.

4. The computer-implemented method of claim 1, wherein the probability of matching a matched real sample record to an individual is the probability of at least one of matching individuals in the population to the real sample record and matching the real sample record to individuals in the population.

5. The computer-implemented method of claim 1, wherein the identity disclosure risk for the synthetic sample is determined according to:

$$max\left(\frac{1}{N}\sum_{s=1}^{n}\frac{1}{f_s}\times I_s\times R_s, \frac{1}{n}\sum_{s=1}^{n}\frac{1}{F_s}\times I_s\times R_s\right).$$

6. The computer-implemented method of claim 1, wherein the identity disclosure risk for the synthetic sample is determined according to one of:

$$\lambda_{mid} \times max \left( \frac{1}{N} \sum_{s=1}^{n} \frac{1}{f_s} \times I_s \times R_s, \frac{1}{N} \sum_{s=1}^{n} \frac{1}{F_s} \times I_s \times R_s \right);$$

and

$$max \left( \frac{1}{N} \sum_{s=1}^{n} \frac{1}{f_s} \times \lambda_{mid} \times I_s \times R_s, \frac{1}{N} \sum_{s=1}^{n} \frac{1}{F_s} \times \lambda_{mid} \times I_s \times R_s \right);$$

where $\lambda_{mid}$ adjusts a probability of a correct match assuming perfect information by an attacker and is based on a verification rate of matches and an error rate of data.

7. The computer-implemented method of claim 1, wherein determining a match between synthetic records and real sample records uses hierarchical or other form of generalization of quasi-identifier variables.

8. The computer-implemented method of claim 7, wherein the determining a match between synthetic records and real sample records uses a generalization lattice, wherein after computing a match at a node in the generalization lattice, unmatched records of the real sample are removed from further matching.

9. The computer-implemented method of claim 7, wherein determining a match between synthetic records and real sample records uses a subset lattice for matching on a subset of quasi-identifier values.

10. The computer-implemented method of claim 1, further comprising:
determining if new information is learned by matching the matched real sample records to individuals.

11. The computer-implemented method of claim 9, wherein the identity disclosure risk is further based on the determination of if new information is learned.

12. A computer-implemented method of determining matches between records in two datasets, the method comprising:

generating generalization lattice of quasi-identifier variables used for matching records in the first dataset to records in the second dataset, wherein each node of the generalization lattice uses a generalization of at least one of the quasi-identifier variables;
processing each node of the generalization lattice to determine if any of the records in the first dataset match records in the second dataset using the generalizations of the lattice node for the quasi-identifier variables;
after processing each node, removing from further node processing any records in the second dataset that were not matched,
wherein the lattice nodes are processed from a broadest generalization to a narrowest generalization.

13. The computer-implemented method of claim 12, wherein determining matches between records in two datasets further comprises:
using a subset lattice wherein each node comprises respective subsets of quasi identifier variables.

14. The computer-implemented method of claim 13, wherein each node in the subset lattice is processed using a respective generalization lattice using the subset of quasi-identifiers of the node as the quasi-identifiers of the generalization lattice.

15. A non-transitory computer readable media storing instructions which when executed by a processor perform a method according to any one of claims 1 to 14.

102

1

| Population (P) |

N

sampling

Matching between records
in the synthetic sample and
individuals in the population

1

| Real Sample (R) |

n

synthesis

1

| Synthetic Sample (S) |

n

104

106

**FIG.  1**

**FIG. 2**

<u>300</u>

302 — **Determine identity matching risk**

302a — Determine population to synthetic sample matching risk

Determine synthetic sample to population matching risk — 302b

302c

304 — Determine new information gain

306 — Determine meaningful information disclosure risk

**FIG. 3**

400

```
          ┌─────────────────────────┐
          │   Determine if there is a │
  402 ∿───┤  match between a synthetic│
          │  record(s) and real record(s)│
          └───────────┬─────────────┘
                      │
                      ▼
          ┌─────────────────────────┐
          │  Determine possibility of │
  404 ∿───┤  matching real record(s) to│
          │       individual(s)       │
          └───────────┬─────────────┘
                      │
                      ▼
          ┌─────────────────────────┐
          │    Determine identity     │
  406 ∿───┤    disclosure risk level  │
          │                           │
          └───────────┬─────────────┘
                      │
                      ▼
          ┌─────────────────────────┐
          │     Determine new         │
  408 ∿───┤  information disclosure risk│
          │                           │
          └─────────────────────────┘
```

FIG.    4

500

```
                    ┌──────────────────┐   ┌──────────────────┐
502  ∼              │ Receive real data│   │Receive synthetic │            ∼  504
                    │                  │   │      data        │
                    └──────────────────┘   └──────────────────┘
                             │                      │
                             └──────────┬───────────┘
                                        ▼
                            ┌──────────────────────┐
                            │  For each data record│
506  ∼                      │   of synthetic data  │
                            └──────────────────────┘
                                        │
                                        ▼
                            ┌──────────────────────┐
                            │ Determine identity   │
508  ∼                      │  disclosure level    │
                            └──────────────────────┘
                                        │
                                        ▼
                            ┌──────────────────────┐
510  ∼                      │   Next data record   │
                            └──────────────────────┘
                                        │
                                        ▼
                            ┌──────────────────────┐
512  ∼                      │Output identity       │
                            │disclosure levels     │
                            └──────────────────────┘
```

FIG.    5

600

602 — Receive real sample data

604 — Create synthesizer model from real sample data

606 — Generate synthetic data using synthesizer

608 — For each data record

610 — Determine identity disclosure level

612 — Identity disclosure level acceptable?

No → 616b Adjust record value(s)
616a Remove record from synthetic data

Yes

614 — Next data record

618 — Synthetic data profile matches real data profile?

→ Adjust synthesizer parameters

622

620 — Output safe synthetic data

**FIG. 6**

$t_2$  years
$t_1$  months
$t_0$  weeks

(A)

$b_1$  underweight, normal, obese
$b_0$  exact

(B)

$s_4$  years
$s_3$  quarters
$s_2$  months
$s_1$  weeks
$s_0$  days

(C)

FIG.   7

800

802

$<t_2,b_1,s_4>$

$<t_1,b_1,s_4>$    $<t_2,b_0,s_4>$    $<t_2,b_1,s_3>$

806    808    810

$<t_0,b_1,s_4>$    $<t_1,b_0,s_4>$    $<t_1,b_1,s_3>$    $<t_2,b_0,s_3>$    $<t_2,b_1,s_2>$

812

$<t_0,b_0,s_4>$    $<t_0,b_1,s_3>$    $<t_1,b_0,s_3>$    $<t_1,b_1,s_2>$    $<t_2,b_0,s_2>$    $<t_2,b_1,s_1>$

$<t_0,b_0,s_3>$    $<t_0,b_1,s_2>$    $<t_1,b_0,s_2>$    $<t_1,b_1,s_1>$    $<t_2,b_0,s_1>$    $<t_2,b_1,s_0>$

$<t_0,b_0,s_2>$    $<t_0,b_1,s_1>$    $<t_1,b_0,s_1>$    $<t_1,b_1,s_0>$    $<t_2,b_0,s_0>$

$<t_0,b_0,s_1>$    $<t_0,b_1,s_0>$    $<t_1,b_0,s_0>$

$<t_0,b_0,s_0>$

804

# FIG. 8

900

FIG. 9

Europäisches
Patentamt

European
Patent Office

Office européen
des brevets

# EUROPEAN SEARCH REPORT

Application Number

EP 21 16 9416

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HU JINGCHEN ET AL: "Disclosure Risk Evaluation for Fully Synthetic Categorical Data", 17 September 2014 (2014-09-17), ICIAP: INTERNATIONAL CONFERENCE ON IMAGE ANALYSIS AND PROCESSING, 17TH INTERNATIONAL CONFERENCE, NAPLES, ITALY, SEPTEMBER 9-13, 2013. PROCEEDINGS; [LECTURE NOTES IN COMPUTER SCIENCE; LECT.NOTES COMPUTER], SPRINGER, BERLIN, HEIDELBERG, PAGE(S) 185 - 1, XP047299069, ISBN: 978-3-642-17318-9 section 3; * abstract * | 1-15 | INV. G06F17/18 G06N3/04 G06N5/00 G16H50/00 G06F21/00 |
| | ----- | | |

| TECHNICAL FIELDS SEARCHED (IPC) |
|---|
| G06F G16H |

The present search report has been drawn up for all claims

| Place of search | Date of completion of the search | Examiner |
|---|---|---|
| Berlin | 7 September 2021 | Virnik, Elena |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   .....................................................................................
& : member of the same patent family, corresponding
   document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K. El.EMAM.** Guide to the De-Identification of Personal Health Information. CRC Press, 2013